# EUROPEAN PATENT APPLICATION

(11) **EP 2 873 428 A1**
(43) Date of publication of application: **20.05.2015**
(21) Application number: 13816703.6
(22) Date of filing: 30.05.2013
(51) Int. Cl.: A61L 27/00, A61F 2/14

(54) **SUBSTRATE FOR FORMATION OF MEMBRANE-LIKE CONNECTIVE TISSUE AND PRODUCTION METHOD FOR MEMBRANE-LIKE CONNECTIVE TISSUE USING SAME**

(30) Priority: 11.07.2012 JP 2012155334
(71) Applicant: National Cerebral and Cardiovascular Center, Suita-shi Osaka 565-8565 (JP)
(72) Inventor: NAKAYAMA,Yasuhide, Suita-shi Osaka 565-8565 (JP); OIE,Tomonori, Osaka-shi Osaka 555-0011 (JP)
(74) Representative: Müller Hoffmann & Partner
(86) International application number: PCT/JP2013/064999
(87) International publication number: WO 2014/010323

(57) **Abstract**

The present invention provides a membranous connective tissue-forming substrate on which a membranous connective tissue having a sufficient thickness and strength can be formed and a method for producing a membranous connective tissue by using the substrate. Specifically, a connective tissue-forming section 2 is provided on the surface of the substrate. The connective tissue-forming section 2 is surrounded by a thickness-increasing section 3 to enlarge a membranous connective tissue-forming substrate 1 as a whole. The membranous connective tissue-forming substrate 1 is placed under an environment in which a biological tissue material is present to form a membranous connective tissue on the surface of the substrate. In proportion as the size of the membranous connective tissue-forming substrate 1 increases, the thickness of the membranous connective tissue increases. A surplus portion formed on the surface of the thickness-increasing section 3 is removed from the connective tissue. A membranous connective tissue having an increased thickness and strength can be thus obtained.

## Description

### Technical Field

The present invention relates to a membranous connective tissue-forming substrate used to form a membranous connective tissue such as an artificial cornea and to a method for producing a membranous connective tissue by using the substrate.

### Background Art

Many kinds of research on regenerative medicine have been carried out, including using artificial materials and cells to regenerate cells, tissues or organs that have been lost due to diseases or accidents.

In general, a human body has a self-defense mechanism. When a foreign material such as a thorn invades into a shallow site of the body, the mechanism tries to eject the material outside the body. It is also known that when a foreign material invades into a deep site of the body, fibroblasts gradually accumulate around the material to form a connective tissue capsule mainly containing fibroblasts and collagen thereby to cover the foreign material, resulting in the foreign material being separated by the capsule *in vivo.* Some have reported techniques of implanting a foreign substrate *in vivo* to form a connective tissue, which uses the latter self-defense reaction to form a biological tissue derived from living cells (see Patent Literatures 1 to 3).

When such a method is used to form a connective tissue, the shape and size of the foreign substrate to be implanted *in vivo* can be appropriately set. This enables the connective tissue to be formed at a desired shape and size.

### Citation List

### Patent Literature

Patent Literature 1
   Japanese Patent Laid-Open No. 2007-312821
Patent Literature 2
   Japanese Patent Laid-Open No. 2008-237896
Patent Literature 3
   Japanese Patent Laid-Open No. 2010-094476

### Summary of Invention

### Technical Problem

When a connective tissue is formed to surround a substrate implanted *in vivo,* the connective tissue having a desired shape and size can be formed in the periphery of the substrate by appropriately setting the shape and size of the substrate. However, the connective tissue may not have a desired thickness and strength. Consequently, a connective tissue having a sufficient thickness and strength cannot always be obtained.

It is an object of the present invention to provide a membranous connective tissue-forming substrate on which a membranous connective tissue having a sufficient thickness and strength can be formed and to provide a method for producing a membranous connective tissue by using the substrate.

### Solution to Problem

To achieve the above objective, a membranous connective tissue-forming substrate according to the present invention can form a membranous connective tissue on a surface of the substrate when being placed under an environment in which a biological tissue material is present, and comprises a connective tissue-forming section provided on the surface of the substrate, for forming a membranous connective tissue, and a thickness-increasing section surrounding the connective tissue-forming section, for increasing a thickness of the connective tissue.

According to the above configuration, since the connective tissue-forming section provided on the surface of the substrate is surrounded by the thickness-increasing section, the thickness of the connective tissue formed on the surface of the substrate can be increased. Also, this configuration can increase not only the thickness of the membranous connective tissue but also its strength, compared to the case of having only the connective tissue-forming section on the surface of the substrate.

More specifically, the research has been focused on the size of the substrate as a factor affecting the thickness of the membranous connective tissue, and has examined the relationship between the thickness of the membranous connective tissue and the size of the substrate used to form this membranous connective tissue. Then it has been found that in proportion as the size of the substrate increases, not only the thickness of the connective tissue formed on the surface of the substrate but also its strength increases.

Accordingly, a connective tissue-forming section having an area required to form a membranous connective tissue with a desired size is provided on the surface of the substrate, and in addition, a thickness-increasing section is provided on the periphery of the connective tissue-forming section. This configuration enlarges the area of the surface of the substrate as a whole. Because the size of the substrate is increased as a whole, this configuration enables a connective tissue having an increased thickness and strength to be formed on its surface. Then, by removing a surplus portion formed on the surface of the thickness-increasing section surrounding the connective tissue-forming section, a membranous connective tissue having an increased thickness and strength can be obtained.

As used herein, the term "biological tissue material" refers to a substance necessary to form a desired tissue derived from a living organism. Examples of the material include: animal cells such as fibroblasts, smooth muscle cells, endothelial cells, stem cells, ES cells, and iPS cells; various proteins (collagen, elastin); saccharides such as hyaluronic acid; and various physiological active substances present *in vivo,* such as cell growth factors and cytokines. Examples of the "biological tissue material" include: those derived from mammals such as a human, dog, cow, pig, goat, and sheep, birds, fish, and other animals; and equivalent artificial materials.

In addition, the term "under an environment in which a biological tissue material is present" refers to an *in vivo* environment (e.g., subcutaneously implanted in the limbs, lumber, back, or abdomen; or intraperitoneally implanted) of animals (mammals such as a human, dog, cow, pig, goat, and sheep, birds, fish, other animals) or an ex *vivo* artificial environment containing a biological tissue material.

The membranous connective tissue-forming substrate of the present invention can be used to form a membranous connective tissue for any usage. Use of the configuration of the present invention is particularly suitable for a substrate that is used as an artificial cornea-forming substrate used to form an artificial cornea as a connective tissue. That is, a membranous connective tissue formed by the substrate of the present invention is used as a flat membranous tissue that covers a surface layer or functions in a membranous form, and examples of the membranous tissue may include any kinds such as a pericardium, dura mater, skin, and valve. It is hard to form a thick tissue as a cornea because its size is small, and accordingly, it is preferable to use the substrate of the present invention to increase not only the thickness of an artificial cornea but also its strength.

In addition, the present invention provides a method for producing a membranous connective tissue by using the above membranous connective tissue-forming substrate. Specifically, a method for producing a membranous connective tissue according to the present invention comprises: a placement step of placing the membranous connective tissue-forming substrate under an environment in which a biological tissue material is present; a formation step of forming a connective tissue on a periphery of the membranous connective tissue-forming substrate; a pick-up step of picking up the membranous connective tissue-forming substrate covered with the connective tissue from the environment; a separation step of peeling, for separating, the connective tissue from the membranous connective tissue-forming substrate; and a removal step of removing a surplus portion surrounding the membranous connective tissue from the connective tissue.

This configuration can achieve the same effect as in the configuration of the above membranous connective tissue-forming substrate. Note that in the present invention, any of an autograft, allograft, and xenograft to a transplant recipient may be adopted. In view of avoiding rejection, however, the autograft or allograft is preferable. Also, in the case of the xenograft, it is preferable to perform a known immunogen removal procedure such as decellularization so as to avoid the rejection.

In addition, the present invention provides a membranous connective tissue formed by using a larger substrate to form a connective tissue with a sufficient thickness and removing a surrounding surplus portion from the connective tissue, as described in the above production method. Specifically, the present invention provides a membranous connective tissue formed on a surface of a substrate under an environment in which a biological tissue material is present, wherein a surplus portion surrounding the membranous connective tissue has been removed.

### Advantageous Effects of Invention

As described above, according to the present invention, a thickness-increasing section is provided on the periphery of a connective tissue-forming section to make the substrate larger than a membranous connective tissue produced by using the substrate. Consequently, the thickness of the connective tissue formed on the surface of the substrate can be increased as well as its strength. Then, a surrounding surplus portion can be removed to produce a membranous connective tissue having an increased thickness and strength. Even if a small membranous connective tissue such as a cornea is formed, a sufficient thickness and strength can be achieved.

### Brief Description of Drawings

[Figure 1] Figure 1 is a perspective view of a membranous connective tissue-forming substrate according to the present invention.
[Figure 2] Figure 2 includes photographs each showing a connective tissue formed on the surface of a cylindrical substrate, and (a), (b) and (c) show a case of a cylindrical substrate with a diameter of 10 mm, 20 mm and 30 mm, respectively.
[Figure 3] Figure 3 includes photographs each showing a cross section of a connective tissue formed on the surface of a cylindrical substrate, and (a), (b), (c) and (d) show a case of a cylindrical substrate with a diameter of 2 mm, 10 mm, 20 mm and 30 mm, respectively.
[Figure 4] Figure 4 includes enlarged photographs of (a) to (d) of Figure 3.
[Figure 5] Figure 5 is a graph illustrating the relationship between the axial length of a cylindrical substrate and the thickness of a connective tissue.
[Figure 6] Figure 6 is a graph illustrating the relationship between the diameter of an artificial cornea-forming substrate and the thickness of a connective tissue.
[Figure 7] Figure 7 is a perspective view showing a semicylindrically-shaped membranous connective tissue-forming substrate.
[Figure 8] Figure 8 is a perspective view showing a sheet-shaped membranous connective tissue-forming substrate.
[Figure 9] Figure 9 is a graph illustrating the relationship between the side length of a square substrate and the thickness of a connective tissue.

### Description of Embodiments

With reference to the drawings, embodiments of the membranous connective tissue-forming substrates and the method for producing a membranous connective tissue by using the substrate according to the present invention will be described below.

A membranous connective tissue-forming substrate 1 is, for example, placed under an environment in which a biological tissue material is present to form a membranous connective tissue used as an artificial cornea on the surface of the substrate. The artificial cornea-forming substrate comprises: a connective tissue-forming section 2 provided on the surface of the substrate, for forming a membranous connective tissue; and a thickness-increasing section 3 surrounding the connective tissue-forming section 2, for increasing a thickness of the connective tissue.

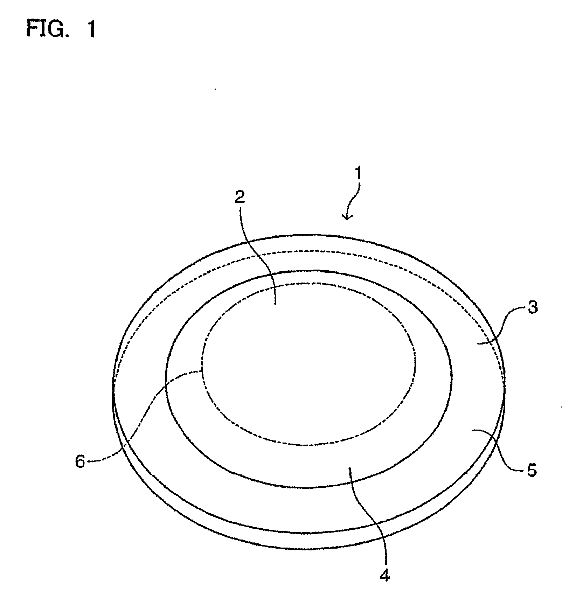

As shown in Figure 1, the membranous connective tissue-forming substrate 1 is a disk including: a convex section 4 building out spherically and convexly in its center region on one surface; and a flat marginal section 5 surrounding the convex section 4. The center region of the convex section 4 serves as a connective tissue-forming section 2, and the marginal section 5 and the rest of the convex section 4 serve as a thickness-increasing section 3. Note that in Figure 1, the two-dot chain line is a virtual line denoting an interface 6 between the connective tissue-forming section 2 and the thickness-increasing section 3.

The shape and size of the connective tissue-forming section 2 are determined depending on the shape and size of an artificial cornea to be formed. The overall size of the membranous connective tissue-forming substrate 1 having the thickness-increasing section 3 is determined depending on the thickness of the artificial cornea to be formed. Here, the size of the membranous connective tissue-forming substrate 1 can be defined by an area contacting an environment in which a biological tissue material is present, namely the surface area of the membranous connective tissue-forming substrate 1.

A material for the membranous connective tissue-forming substrate 1 is not particularly limited, but is preferably a resin meeting conditions that it has a sufficient strength (hardness) to prevent deformation caused by *in vivo* implantation, chemical stability, and resistance to load such as sterilization, and also that it discharges no or little living body-stimulating effluent. Examples of the resin may include a silicone resin and an acrylic resin. In addition, when the membranous connective tissue-forming substrate 1 is made of a highly elastic material, the thickness of a connective tissue formed on its surface is likely to increase. Thus, it is more preferable to prepare at least the surface of the membranous connective tissue-forming substrate 1 with an elastomer such as a silicone resin.

A method for producing a membranous connective tissue by using the above membranous connective tissue-forming substrate 1 will be described below.

This production method comprises: a "placement step" of placing a membranous connective tissue-forming substrate 1 under an environment in which a biological tissue material is present; a "formation step" of forming a connective tissue on a periphery of the membranous connective tissue-forming substrate 1; a "pick-up step" of picking up the membranous connective tissue-forming substrate 1 covered with the connective tissue from the environment; a "separation step" of peeling, for separating, the connective tissue from the membranous connective tissue-forming substrate 1; and a "removal step" of removing a surplus portion surrounding a membranous connective tissue as an artificial cornea from the connective tissue.

### <Placement Step>

A membranous connective tissue-forming substrate 1 is placed under an environment in which a biological tissue material is present. Examples of the environment in which a biological tissue material is present include an *in vivo* environment (e.g., subcutaneous and/or intraperitoneal implantation) and an ex *vivo* artificial environment such as a solution containing a floating biological tissue material. Examples of the biological tissue material that can be used include: materials derived from mammals such as a human, dog, cow, pig, goat, rabbit, and sheep; materials derived from birds, fish, and other animals; and artificial materials.

When the membranous connective tissue-forming substrate 1 is implanted in an animal, minimum surgery is performed under sufficient anesthesia. After the implantation, the incision is closed with a suture. Preferable sites for the implantation of the membranous connective tissue-forming substrate 1 include, for example, an intraperitoneal region having an enough volume to receive the membranous connective tissue-forming substrate 1 and a subcutaneous region of the limbs, shoulder, back, or abdomen. In addition, when the membranous connective tissue-forming substrate 1 is placed under an environment in which biological tissue material is present, culture of cells may be carried out while adjusting various culture conditions under a clean environment in accordance with a known procedure.

### <Formation Step>

By the passage of a predetermined time following the placement step, a membranous connective tissue is formed on the periphery of the membranous connective tissue-forming substrate 1. The connective tissue includes an extracellular matrix such as fibroblasts and collagen, and is formed to have a sufficient thickness depending on the size of the membranous connective tissue-forming substrate 1.

### <Pick-up Step>

After the connective tissue is sufficiently formed through a predetermined time of the formation step, a pick-up step of picking up the membranous connective tissue-forming substrate 1 from the environment in which a biological tissue material is present is carried out. The whole membranous connective tissue-forming substrate 1 that has been picked up from the environment in which a biological tissue material is present is covered with a membrane of connective tissue.

### <Separation Step>

The connective tissue covering a marginal section 5 of the membranous connective tissue-forming substrate 1 is removed and the membrane of connective tissue covering a convex section 4 is peeled. This membrane of connective tissue has a size and shape corresponding to the size and curvature of the convex section 4, and is formed to have a sufficient thickness depending on the size of the membranous connective tissue-forming substrate 1.

### <Removal Step>

Then, a surplus portion on the marginal section is removed from the connective tissue membrane that has been peeled off from the convex section 4 to obtain an artificial cornea with a desired size.

When a produced artificial cornea is transplanted as a xenograft, an immunogen removal procedure such as decellularization, dehydration, and fixation is preferably performed so as to avoid rejection after the transplantation. Examples of the decellularization include sonication, detergent treatment, and a washing process in which an extracellular matrix is eluted by treatment using an enzyme such as collagenase. Examples of the dehydration include washing processes using a water-soluble organic solvent such as methanol, ethanol, and isopropyl alcohol. Examples of the fixation include processes using an aldehyde compound such as glutaraldehyde and formaldehyde.

According to the above configuration, the connective tissue-forming section 2 is surrounded by the thickness-increasing section 3, which increases the size of the membranous connective tissue-forming substrate 1. This allows a connective tissue to be formed at a sufficient thickness depending on the size. Accordingly, a surplus portion on its marginal section can be removed from the connective tissue membrane formed so as to have a sufficient thickness to obtain an artificial cornea with a desired size, curvature, and a sufficient thickness.

Hereinafter, as for the relationship between the size of a substrate and the thickness of a connective tissue formed on the surface of the substrate, "Relationship between Diameter of Cylindrical Substrate and Thickness of Connective Tissue", "Relationship between Axial Length of Cylindrical Substrate and Thickness of Connective Tissue", and "Relationship between Diameter of Substrate Having Convex Section and Thickness of Connective Tissue" will be described in this order.

### <Relationship between Diameter of Cylindrical Substrate and Thickness of Connective Tissue>

First, four kinds of a silicone cylindrical substrate were subcutaneously implanted in the back of a beagle dog whose body weight was 10 kg. Next, the beagle dog was kept under conventional conditions for 1 month. Then, the cylindrical substrates covered with a connective tissue were removed. The cylindrical substrates are made of sterile silicone, and all have an axial length of 30 mm. The diameters of them are four kinds: 2 mm, 10 mm, 20 mm, or 30 mm.

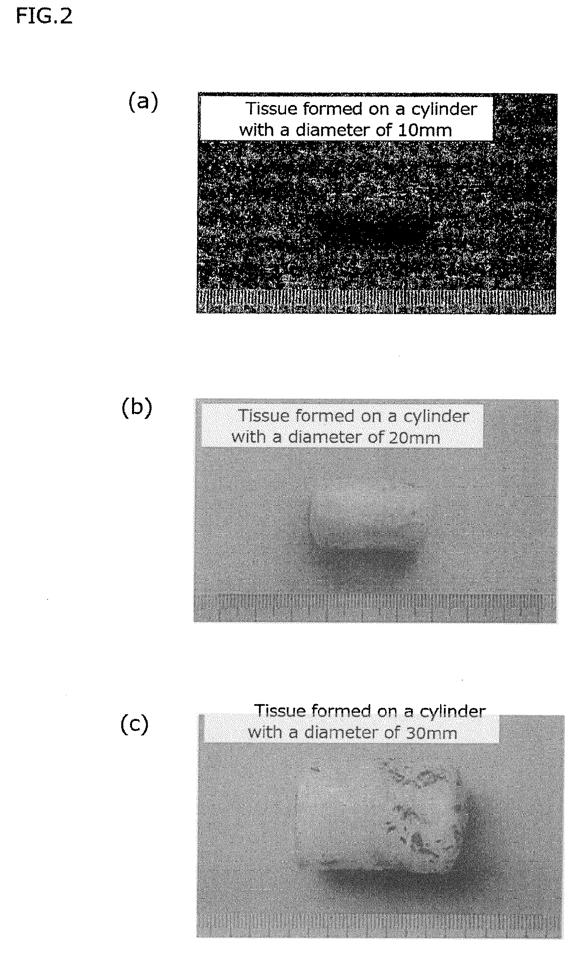

Figure 2 includes photographs each showing appearance of the three kinds of the cylindrical substrate with a diameter of 10 mm, 20 mm, or 30 mm when covered with a connective tissue. The subcutaneously formed connective tissue mainly contained fibroblasts and collagen, and encapsulated the cylindrical substrates to cover the surface of the substrate therewith. In addition, the cylindrical substrates covered with the membrane of connective tissue were successfully and easily removed from a subcutaneous tissue.

Next, a connective tissue was peeled off and separated from the surface of the substrate. Then, this connective tissue was cut to examine its thickness. Note that the cylindrical substrate did not adhere to the surrounding connective tissue at all, and that both were successfully and easily separated from each other without damage.

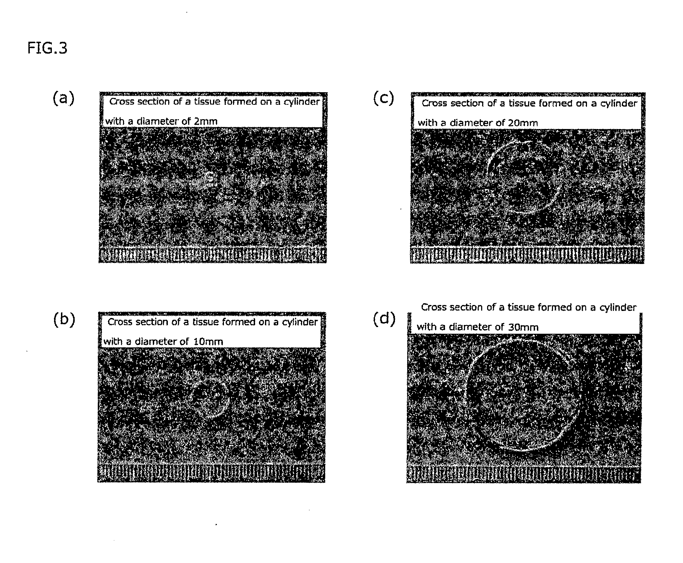

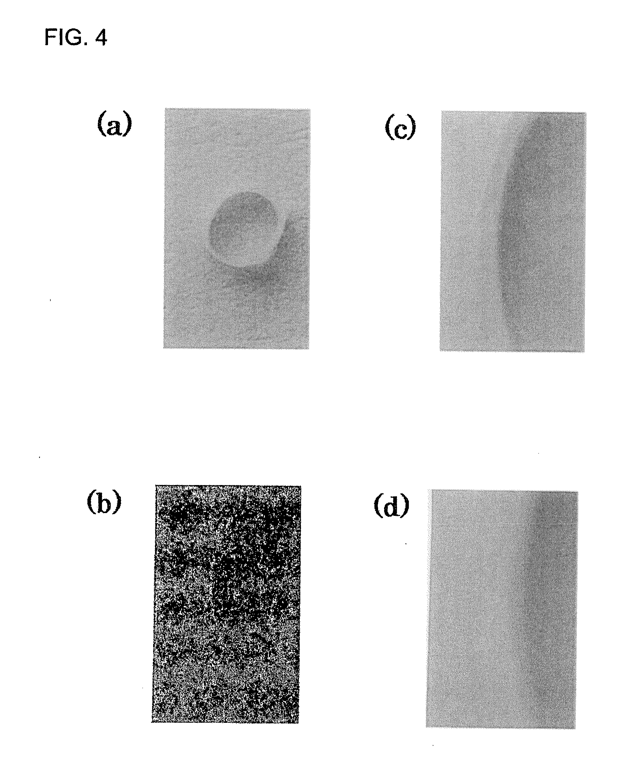

Regarding four kinds of the cylindrical substrate with a diameter of 2 mm, 10 mm, 20 mm, or 30 mm, Figure 3 includes photographs each showing a cross section of the connective tissue separated from the surface of the substrate. Figure 4 includes their enlarged photographs. When the cylindrical substrate had a diameter of 2 mm, the connective tissue had a thickness of 85 ± 27 µm; in the case with a diameter of 10 mm, the connective tissue had a thickness of 237 ± 55 µm; in the case with a diameter of 20 mm, the connective tissue had a thickness of 374 ± 128 µm; and in the case with a diameter of 30 mm, the connective tissue had a thickness of 813 ± 175 µm.

### <Relationship between Axial Length of Cylindrical Substrate and Thickness of Connective Tissue>

Four kinds of a cylindrical substrate, whose diameter was 10 mm and axial length was 1 mm, 10 mm, 30 mm, or 50 mm, were used. A membrane of connective tissue was formed under the same condition as in <Relationship between Diameter of Cylindrical Substrate and Thickness of Connective Tissue> described above. Then, its thickness was measured.

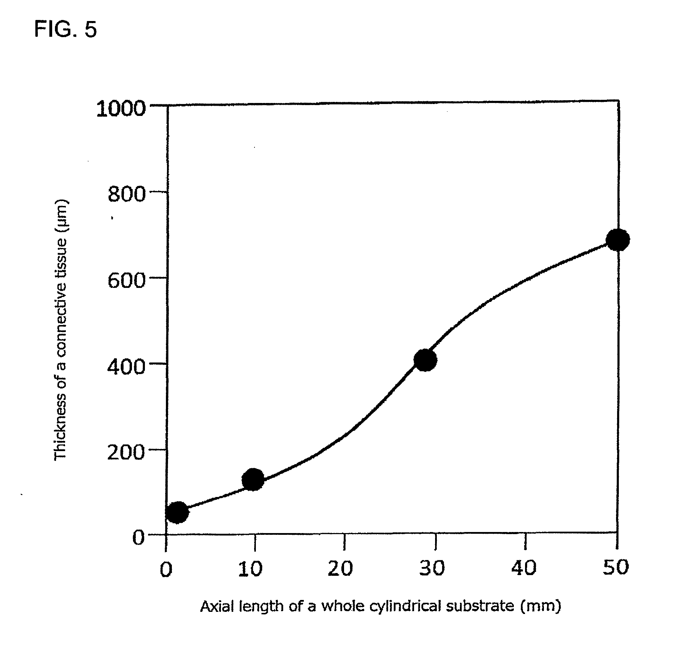

Figure 5 demonstrates that when the cylindrical substrate had an axial length of 1 mm, the connective tissue had a thickness of 100 µm or less, but that as the axial length of the cylindrical substrate increased, the thickness of the connective tissue increased.

### <Relationship between Diameter of Substrate Having Convex Section and Thickness of Connective Tissue>

The diameter of the connective tissue-forming section 2 was set to 7 mm, which was a cornea size required for transplantation. Then, a total of five kinds of a substrate were used, including a substrate including only the connective tissue-forming section 2 and the four membranous connective tissue-forming substrates 1 including the rest of the convex section 4 and the marginal section 5 in addition to the connective tissue-forming section 2, the four substrates having a diameter of 10 mm, 20 mm, 30 mm, or 50 mm. A connective tissue membrane was formed under the same condition as in <Relationship between Diameter of Cylindrical Substrate and Thickness of Connective Tissue> described above. Then, its thickness was measured. Note that all five substrates are made of acryl, and the marginal section 5 has a thickness of 5 mm.

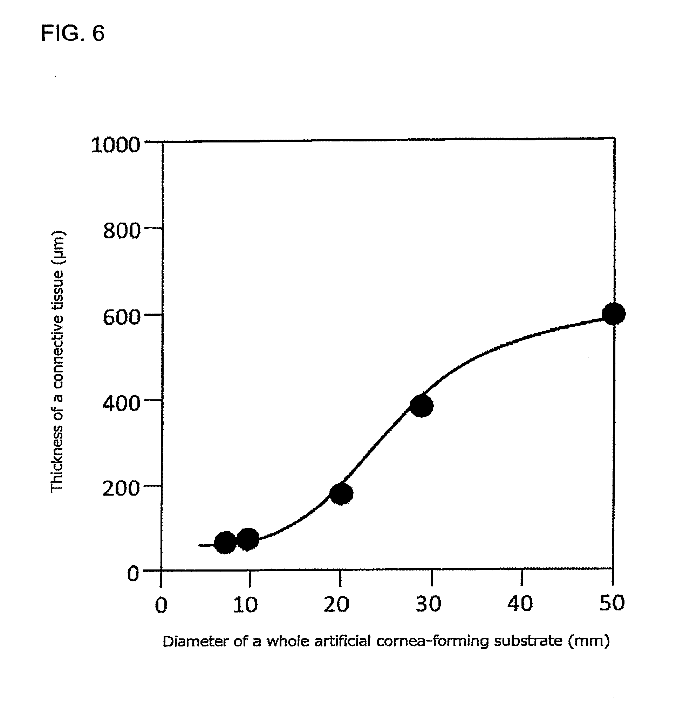

Figure 6 demonstrates that when the substrate including only the connective tissue-forming section 2 has a diameter of 7 mm, the thickness is 100 µm or less, but that in proportion as the diameter of the whole substrate increases to 20 mm or 30 mm, the thickness of the connective tissue increases. That is, it is demonstrated that in proportion as the diameter of the substrate increases, the connective tissue having a thicker membrane is obtained.

Corneas with a diameter of about 7 mm and a thickness of about 0.5 mm have been used in keratoplasty. Corneas with a diameter of about 7 mm and a thickness of 0.2 mm to 0.4 mm have been used in nonpenetrating keratoplasty. Thus, when the substrate including only the connective tissue-forming section 2 has a diameter of 7 mm, an artificial cornea having a thickness required for transplantation is unable to be formed. When the diameter of the whole substrate is 20 mm, an artificial cornea having a thickness sufficient for nonpenetrating keratoplasty is able to be formed. When the diameter of the whole substrate is 30 mm or more, an artificial cornea having a thickness sufficient for penetrating keratoplasty is able to be formed.

In this way, it has been demonstrated that a thicker connective tissue is obtained in proportion as the diameter of the cylindrical substrate, the axial length of the cylindrical substrate, or the diameter of the substrate having a convex section increases. That is, it has been demonstrated that although the shape of the substrate makes a difference, the substrate produces a thicker connective tissue in proportion as its surface area increases with the same shape.

Note that the present invention is not limited to the above embodiments. Appropriate modifications can be added within the scope of the invention. For example, a membranous connective tissue produced using the membranous connective tissue-forming substrate 1 may be a flat tissue that covers a surface layer or functions in a membranous form. The membranous connective tissue is not limited to an artificial cornea, but can be used as a pericardium, dura mater, skin, valve, or the like.

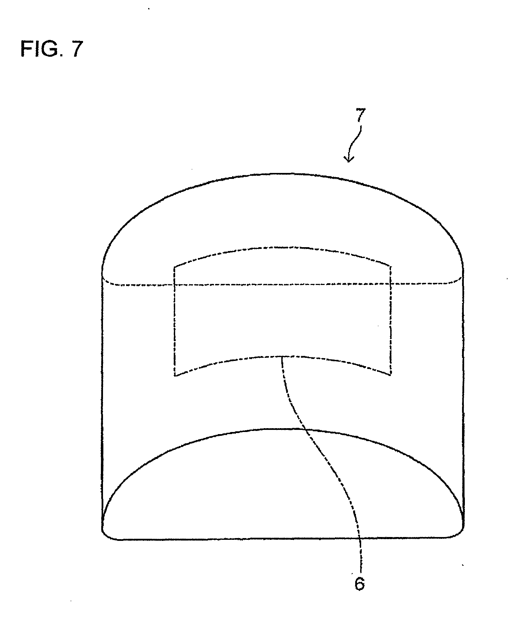

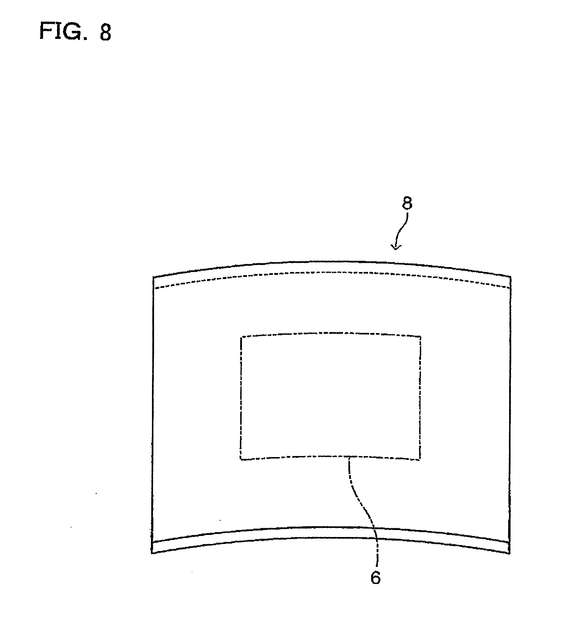

In addition, examples of the membranous connective tissue-forming substrate are not limited to those having a convex section 4 building out spherically and convexly in its center region, and a semicylindrically-shaped substrate 7 (see Figure 7) or a sheet-shaped substrate 8 (see Figure 8) can be employed depending on the shape of a membranous connective tissue to be produced. Note that the semicylindrically-shaped substrate 7 or the sheet-shaped substrate 8 with a large size can produce a connective tissue with a sufficient thickness similarly to the above cylindrical substrate and the convex section-containing substrate.

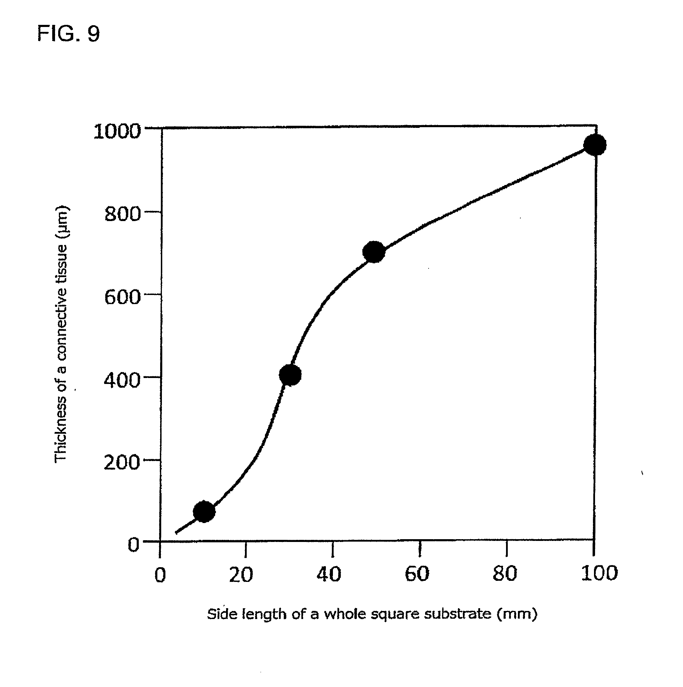

Figure 9, for example, indicates the relationship between the size of a sheet-shaped substrate and the thickness of a connective tissue. The sheet-shaped substrates 8 were four kinds, and they all had a thickness of 1 mm and were square. The length of the side was 10 mm, 30 mm, 50 mm, or 100 mm. The substrates were made of acryl. A connective tissue membrane was formed under the same condition as of the above cylindrical substrate. Then, its thickness was measured. When the substrate had a side length of 10 mm, the connective tissue formed on its surface had a thickness of 100 µm or less. However, when the substrate had a side length of 30 mm, the connective tissue had a thickness of about 400 µm. When the substrate had a side length of 60 mm, the connective tissue had a thickness of about 700 µm. When the substrate had a side length of 100 mm, the connective tissue had a thickness of about 1000 µm. Accordingly, in proportion as the size of the substrate increased, the thickness of the membrane increased.

### Reference Signs List

1 Membranous connective tissue-forming substrate
2 Connective tissue-forming section
3 Thickness-increasing section
4 Convex section
5 Marginal section
6 Interface
7 Substrate (semicylindrically-shaped)
8 Substrate (sheet-shaped)

## Claims

1. A membranous connective tissue-forming substrate,
wherein the substrate can form a membranous connective tissue on a surface of the substrate when being placed under an environment in which a biological tissue material is present; and
the substrate comprises:
a connective tissue-forming section provided on the surface of the substrate, for forming a membranous connective tissue, and
a thickness-increasing section surrounding the connective tissue-forming section, for increasing a thickness of the connective tissue.

2. The membranous connective tissue-forming substrate according to Claim 1, wherein the substrate is used as an artificial cornea-forming substrate capable of forming an artificial cornea as the connective tissue.

3. A method for producing a membranous connective tissue, comprising:
a placement step of placing the membranous connective tissue-forming substrate according to Claim 1 or 2 under an environment in which a biological tissue material is present;
a formation step of forming a connective tissue on a periphery of the membranous connective tissue-forming substrate;
a pick-up step of picking up the membranous connective tissue-forming substrate covered with the connective tissue from the environment;
a separation step of peeling, for separating, the connective tissue from the membranous connective tissue-forming substrate; and
a removal step of removing a surplus portion surrounding the membranous connective tissue from the connective tissue.

4. A membranous connective tissue formed on a surface of a substrate under an environment in which a biological tissue material is present, wherein a surplus portion surrounding the membranous connective tissue has been removed.
